# FASCICULE DE BREVET EUROPEEN

(11) **EP 4 065 096 B1**
(45) Date de publication et mention de la délivrance du brevet: **22.01.2025**
(21) Numéro de dépôt: 20811372.0
(22) Date de dépôt: 27.11.2020
(51) Int. Cl.: A61K 8/42, A61K 31/164, A61K 36/8968, A61K 9/12, A61P 17/00, A61P 31/04, A61K 8/04, A61K 8/365, A61K 47/20

(54) **COMPOSITIONS VÉTÉRINAIRES NON RINCÉES**
TIERMEDIZINISCHE ZUSAMMENSETZUNGEN OHNE SPUELUNG
NON-RINSED VETERINARY COMPOSITIONS

(30) Priorité: 28.11.2019 EP 19212297
(43) Date de publication de la demande: 05.10.2022
(73) Titulaire: Ceva Santé Animale, 33500 Libourne (FR)
(72) Inventeur: COMMEUREC, Emeline, 33500 LIBOURNE (FR); MAUBERT, Nadège, 33500 LIBOURNE (FR)
(74) Mandataire: Cabinet Becker et Associés
(86) Numéro de dépôt international: PCT/EP2020/083608
(87) Numéro de publication internationale: WO 2021/105340

(56) Documents cités:
- WO-A1-2017/121965
- US-A1- 2015 297 652
- US-A1- 2019 110 967
- UNKNOWN: "DOUXO S3 CALM MOUSSE-ophytrium liquid DOUXO S3 by", FDA, 3 July 2024 (2024-07-03), XP093181520, Retrieved from the Internet <URL:https://fda.report/DailyMed/35a2b189-d05d-4d2e-8a4a-1b93b3d5308b> [retrieved on 20240703]
- UNKNOWN: "DOUXO S3 Calm Mousse for Dogs and Cats Hygiene for Sensitive Skin Relieves and Protects -Hypoallergenic Fragrance 150ml Cream", AMAZON, 3 July 2024 (2024-07-03), XP093181514, Retrieved from the Internet <URL:https://www.amazon.de/-/en/Mousse-Hygiene-Sensitive-Relieves-Protects/dp/B08D6KR64Y/ref=pd_bxgy_thbs_d_sccl_1/258-7561101-2064147?pd_rd_w=WSXP3&content-id=amzn1.sym.d6531279-1f86-4ae1-b1f7-8ab9db04b1a0&pf_rd_p=d6531279-1f86-4ae1-b1f7-8ab9db04b1a0&pf_rd_r=F40H3D4QKYJ22R5ZDG33&pd_rd_wg=W5HqQ&pd_rd_r=9> [retrieved on 20240703]
- EBNER FRITZ ET AL: "Topical use of dexpanthenol in skin disorders", AMERICAN JOURNAL OF CLINICAL DERMATOLOGY, ADIS, US, vol. 3, no. 6, 1 January 2002 (2002-01-01), pages 427 - 433, XP009181863, ISSN: 1175-0561, DOI: 10.2165/00128071-200203060-00005
- UNKNOWN: "Pentavitin binds to keratin in skin to keep the moisture balance", 1 January 2009 (2009-01-01), pages 1 - 2, XP055674420, Retrieved from the Internet <URL:https://www.cosmeticsonline.com.br/subcategorias/SUB19_DSM%20PENTAVITIN.pdf> [retrieved on 20200306]

## Description

### OBJET DE L'INVENTION

La présente invention concerne le domaine vétérinaire et, plus particulièrement, des compositions non rincées comprenant un actif obtenu à partir *d'Ophiopogon japonicus,* du panthénol et du saccharide isomerate destinées aux mammifères non-humains. Elle concerne également un procédé non thérapeutique de traitement du pelage de mammifères non-humains en appliquant sur le pelage et/ou la peau lesdites compositions.

### ARRIERE-PLAN TECHNOLOGIQUE DE L'INVENTION

L'actif obtenu à partir *d'Ophiopogon japonicus* et son effet hydratant ou renforçant la fonction barrière de la peau humaine a été décrit dans le brevet EP2271311. L'utilisation de cet actif pour traiter la dermatite atopique chez les animaux et humains a été décrite dans la demande de brevet WO2017/121965.

La peau et le pelage de mammifères non-humains protègent ces derniers contre les agressions extérieures. Leur entretien est donc très important pour le bien-être, voire la santé, des mammifères non-humains. Il est notamment important de préserver, protéger le bon fonctionnement de la fonction barrière cutanée. Or celle-ci peut être altérée par les agressions extérieures telles que la chaleur, le froid, l'humidité, etc.

Un produit vétérinaire destiné au traitement de la peau ou du pelage doit donc être efficace pour préserver la fonction barrière sans entraîner d'irritation ou toute autre désordre de la peau.

En outre, il existe des bactéries qui peuvent être pathogènes ou devenir pathogènes, si en trop grand nombre, telles que les staphylocoques, pour les chiens et/ou chats qui nécessitent des traitements particuliers en vue de leur réduction ou élimination. Ces staphylocoques sont représentés principalement par S. *pseudintermedius.*

Il existe donc un besoin de compositions pour des mammifères non humains et en particulier des chiens et des chats qui permettent de préserver, voire améliorer, la fonction barrière de la peau et du pelage, et qui confèrent un bon effet hydratant.

Il existe aussi un besoin de préserver la flore bactérienne bénéfique aux mammifères, tout en diminuant, voire éliminant, les bactéries pathogènes.

Il existe aussi un besoin de disposer d'un effet durable par application directe sur la peau ou le pelage des mammifères non humains.

### RESUME DE L'INVENTION

Dans ce contexte, la Demanderesse propose une nouvelle composition vétérinaire non rincée comprenant un actif obtenu à partir *d'Ophiopogon japonicus,* du panthénol et du saccharide isomerate.

La présente invention concerne donc une composition vétérinaire non rincée comprenant un actif obtenu à partir *d'Ophiopogon japonicus*, du panthénol et du saccharide isomerate destinée à être utilisée en tant que produit de traitement pour un mammifère non-humain, en particulier un chien ou un chat.

Un autre objet de l'invention concerne donc un procédé non-thérapeutique pour traiter la peau et/ou le pelage d'un mammifère non-humain, et en particulier un chien ou un chat, en appliquant la composition de la présente invention sur la peau et/ou le pelage dudit mammifère.

Un autre objet de l'invention concerne une composition telle que définie dans la présente demande pour son utilisation pour réduire et/ou prévenir la présence de bactéries pathogènes et/ou la formation et/ou l'adhésion de biofilms de bactéries pathogènes chez un mammifère non-humain, tel que chien ou chat.

### DESCRIPTION DETAILLEE DE L'INVENTION

L'invention telle que décrite dans la présente demande concerne une composition vétérinaire non rincée comprenant un actif obtenu à partir *d'Ophiopogon japonicus* du panthénol et du saccharide isomerate.

Par composition non rincée, on entend une composition qui est formulée pour subsister après application sur la peau ou le pelage des mammifères non-humains. La composition non rincée est appliquée directement sur la peau ou le pelage et ne nécessite pas d'être ensuite éliminée par rinçage à l'eau. La composition non rincée est avantageusement sous forme de mousse.

*L'Ophiopogon japonicus* est une espèce herbacée vivace, basse et foisonnante, à rhizome de la famille des Liliacées. Elle est cultivée comme plante ornementale couvrante à rhizome tubéreux présentant de très nombreuses utilisations en médecine traditionnelle chinoise.

L'espèce est originaire du Japon et de Corée, et est également cultivée au Vietnam et en Chine, notamment dans les provinces du Sichuan, Zhejiang et Hubei. Elle est appelée Muguet du Japon en France, et Mondo grass, Fountain plant, Monkey grass ou Dwarf lilyturf en anglais. La racine tubéreuse a une longueur de quelques centimètres ; elle est de couleur jaune clair à jaune-brun à l'extérieur, avec des rides longitudinales. Son odeur est faible, et son goût légèrement sucré et mucilagineux.

En médecine traditionnelle chinoise, la racine est connue pour « nourrir les Poumons et le Yin, pour nourrir l'Estomac et produire des Fluides, pour éloigner la Chaleur du coeur et pour apaiser l'Esprit ». Les principales indications thérapeutiques concernent les toux sèches, les maux de gorge, l'insomnie, l'irritabilité, la constipation et la diphtérie, selon la 16ème édition de la Pharmacopée Japonaise.

Les racines *d'Ophiopogon japonicus* sont issues de filières cultivées de Chine et de Vietnam. Des extraits *d'Ophiopogon japonicus* sont connus en cosmétique pour la peau, en particulier dans le brevet EP2271311, comme hydratant par action sur le taux de NMF (Natural Moisturizing Factors ou Facteurs naturels d'hydratation) et sur la formation des jonctions serrées des couches cutanées. Il a été également décrit dans la demande WO 2017/121965 que des extraits *d'Ophiopogon japonicus* peuvent être utilisés pour traiter la dermatite atopique.

Par « principe actif » ou « actif « ou « extrait » au sens de l'invention, on entend au moins une molécule, préférentiellement un ensemble de molécules présentant un effet sur les cellules de la peau.

Par « principe actif obtenu à partir *d'Ophiopogon japonicus* » au sens de l'invention on entend toute molécule ou mélange de molécules obtenue(s) à partir *d'Ophiopogon japonicus.* Il peut s'agir de molécules natives de la plante ou de molécules obtenues par tout type de transformation des molécules natives de la plante, par exemple par hydrolyse. Le principe actif selon l'invention est préférentiellement un hydrolysat.

Par « hydrolysat » on entend tout extrait issu *d'Ophiopogon japonicus,* obtenu avec un procédé comprenant au moins une étape d'hydrolyse enzymatique ou chimique *d'Ophiopogon japonicus,* préférentiellement au moins une étape d'hydrolyse enzymatique. Par « *Ophiopogon japonicus* » on entend tout ou partie de la plante. Il peut s'agir de la plante entière ou d'une partie de la plante. Préférentiellement il s'agit de tubercules *d'Ophiopogon japonicus.*

Par « oligosaccharides » on entend des oligomères formés d'un nombre de monosaccharides par liaison glycosidique, le nombre d'unités de monosaccharides étant inférieur à 25 unités. Par « polysaccharides » on entend des polymères constitués de plusieurs oses liés entre eux par des liaisons osidiques, le nombre d'unités de monosaccharides étant supérieur à 25 unités.

Le principe actif obtenu à partir d'*Ophiopogon japonicus* utilisé dans la composition selon l'invention est préférentiellement un principe actif obtenu à partir *d'Ophiopogon japonicus* comprenant des sucres. Encore plus préférentiellement, il comprend des fructosanes, et plus particulièrement il comprend au moins 57% de fructosanes en poids par rapport au poids des sucres totaux du principe actif, encore plus préférentiellement au moins 80%. Les fructosanes sont des polysaccharides composés de fructose et de glucose. Les sucres contenus dans le principe actif sont préférentiellement constitués par 45 à 80% de fructose, par 20 à 50% de glucose et par 0 à 5% de galactose. Ces sucres peuvent être sous forme de monomères, d'oligomères et de polymères. Majoritairement, les sucres contenus dans le principe actif sont des oligo et polysaccharides de poids moléculaires inférieurs à 400 kDa sous forme de fructosanes. Ainsi, préférentiellement le principe actif selon l'invention comprend des oligo et polysaccharides de poids moléculaires inférieurs à 400 kDa sous forme de fructosanes, représentant au moins 57% en poids des sucres présents dans le principe actif. Selon une variante particulièrement adaptée, le principe actif est obtenu à partir de tubercules *d'Ophiopogon japonicus.*

Selon un mode de réalisation, le principe actif utilisé pour préparer la composition selon l'invention se présente sous forme d'une poudre, en particulier une poudre de couleur claire, et présente au moins une des caractéristiques suivantes, préférentiellement toutes:
- un taux de matières sèches compris entre 900 et 1000 mg/g, ou
- une teneur en sucres comprise entre 500 et 800 mg/g, soit au moins 50 % de sucres en poids par rapport au poids de matières sèches.

Le taux de matières sèches peut être mesuré par passage à l'étuve à 105 °C d'un échantillon jusqu'à obtention d'un poids constant.

La teneur globale en sucres peut être déterminée par la méthode de DUBOIS sur une gamme de fructose (Dubois M. et al., Analytical chemistry, 28, 3, 350-356, 1956).

La caractérisation de la masse molaire des carbohydrates présents dans le principe actif de la présente invention peut être réalisée par méthode HPLC et le dosage des sucres simples par chromatographie liquide ionique.

Les masses molaires des carbohydrates sont évaluées par comparaison des temps de rétention des pics détectés dans les échantillons du principe actif avec les temps de rétention de standards injectés au préalable.

Les conditions opératoires sont préférentiellement les suivantes :
- Appareil : HPLC Agilent 1100 Séries
- Colonnes : Colonnes PL aquagel-OH C60, C40, C30 avec pré-colonne de mêmes caractéristiques
- Mode d'élution : isocratique
- Phase mobile : Tampon NaN030.3M + NaH2P04-2H20 0.01M pH7
- Longueurs d'onde de détection : UV 254nm et 280nm

Le principe actif selon l'invention est préférentiellement un principe actif obtenu en milieu aqueux à partir de tubercules *d'Ophiopogon japonicus.* Par « obtenu en milieu aqueux » on entend un milieu contenant principalement de l'eau, ou un milieu aqueux basique ou acide. Notamment il ne s'agit pas d'une huile, ni d'une huile essentielle.

De façon préférée, le principe actif selon l'invention est un hydrolysat *d'Ophiopogon japonicus,* préférentiellement un hydrolysat enzymatique.

Selon une variante particulièrement adaptée, le principe actif selon l'invention est un hydrolysat de tubercules *d'Ophiopogon japonicus,* préférentiellement un hydrolysat enzymatique de tubercules *d'Ophiopogon japonicus.*

En particulier, le principe actif utile selon l'invention, peut être obtenu par la mise en oeuvre des étapes suivantes :
- solubilisation de poudre d'*Ophiopogon japonicus* (préférentiellement de tubercules) dans l'eau à raison d'au moins 50g/l,
- au moins une hydrolyse enzymatique des sucres,
- séparation des phases soluble et insoluble, par exemple par décantation,
- inactivation enzymatique par traitement thermique de la phase soluble.

L'inactivation enzymatique peut être suivie d'une ou plusieurs étapes de filtration(s) et/ou de concentration. Le principe actif peut être obtenu sous forme liquide ou sous forme de poudre par atomisation ou lyophilisation. Préférentiellement il est atomisé, en présence d'un adjuvant d'atomisation type maltodextrine, et utilisé sous forme de poudre.

Les paramètres des différentes étapes doivent être ajustés afin d'obtenir des principes actifs présentant les caractéristiques de l'invention, en particulier la présence de fructosanes présentant un poids moléculaire inférieur à 400 kDa.

Selon un mode de réalisation préféré, le principe actif est atomisé avec de la maltodextrine ; il se présente ainsi sous forme de poudre (avant formulation dans la composition selon l'invention).

La composition selon l'invention comprend avantageusement au moins 0,1 % en poids, par rapport au poids total de la composition, de principe actif obtenu à partir *d'Ophiopogon japonicus,* selon la présente invention, préférentiellement entre 0.1 et 1% en poids, par rapport au poids total de la composition, et plus particulièrement entre 0,2 et 0,6 % en poids, par rapport au poids total de la composition.

La composition selon l'invention comprend du panthénol. La composition comprend avantageusement au moins 0,1 % en poids de panthénol, par rapport au poids total de la composition, préférentiellement de 0,1 % à 1 % en poids, et plus particulièrement de 0,3 % à 0,8 % en poids.

La composition selon l'invention comprend du saccharide isomerate.

Le saccharide isomerate est un actif hydratant. Il contient principalement du fructose et du glucose. La préparation de cet actif est notamment décrite dans le brevet US3578655. Le saccharide isomerate est notamment vendu sous la dénomination « PENTAVITIN^{®} » par la société DSM.

La composition comprend avantageusement au moins 0,1 % en poids de saccharide isomerate, par rapport au poids total de la composition, préférentiellement de 0,1 % à 1 % en poids, et plus particulièrement de 0,3 % à 0,9 % en poids.

De préférence, la composition comprend un ou plusieurs tensioactif(s).

Ledit tensioactif est de préférence choisi parmi les tensioactifs amphotères de type bétaïne, les tensioactifs anioniques carboxyliques, les tensioactifs non ioniques alkyls(poly)glucosides, et leurs mélanges.

Les tensioactifs amphotères de type bétaïne sont de préférence choisis parmi les alkyl(C8-C20)bétaïnes, les alkyl(C8-C20)amidoalkyl(Cl-C8)bétaïnes, les alkyl(C8-C20)amidoalkyl(C1-C8)sulfobétaïnes, les alkyl(C8-C20)amidoalkyl(C1-C6)hydroxysulfobétaïnes, les sulfobetaïnes et les hydroxysulfobétaïnes. A titre d'exemples, on peut citer tout particulièrement les composés classés sous les dénominations Coco-bétaïne, Laury bétaïne, Cetyl-bétaïne, Coco/oleamidopropyl-bétaïne Capryl/capramidopropyl betaïne, Cocamidopropyl-bétaïne, Palmitamidopropyl-bétaïne, Stéaramidopropyl-bétaïne, Cocamidoethyl-bétaïne, Cocamido propyl-hydroxysultaïne, Oleamidopropyl-hydroxysultaïne Coco-hydroxysultaïne, Laurylhydroxysultaïne, Coco-sultaïne, seuls ou en mélanges.

En particulier, ledit au moins un tensioactif amphotère de type bétaïne est choisi parmi les alkyl(C8-C20)amidoalkyl(C1-C6)bétaïnes, les alkyl(C8-C20)amidoalkyl(C1-C6)sulfobétaïnes (appelé aussi alkyl(C8-C20)amidoalkyl(C1-C6)sultaïnes), les alkyl(C8-C20)amidoalkyl(C1-C6)hydroxysulfobétaïnes (appelé aussi alkyl(C8-C20)amidoalkyl(C1-C6)hydroxysultaïnes). De préférence, le tensioactif amphotère de type bétaïne est choisi parmi les alkyl(C8-C20)amidoalkyl(C1-C6)bétaïnes. Il est plus spécifiquement la capryl/capramidopropyl betaïne (notamment vendue par la société Evonik sous le nom commercial « Tego^{®} betaïne 810 »).

De manière plus spécifique, la composition peut comprendre de la capryl/capramidopropyl betaïne. La quantité de capryl/capramidopropyl betaïne peut être comprise entre 0,1 et 5 % en poids, de préférence entre 0,3 et 3 % en poids, par rapport au poids total de la composition.

Selon l'invention, les tensioactifs anioniques carboxyliques sont des tensioactifs anioniques comportant au moins une fonction carboxylique (-COOH) éventuellement sous forme de sel (-COO⁻).

Les tensioactifs anioniques de type carboxyliques peuvent être notamment choisis parmi les acides d'alkyl D galactoside uroniques et leurs sels, les acides alkyl (C6-C24) éther carboxyliques polyoxyalkylénés, les acides alkyl(C6-C24)aryl éther carboxyliques polyoxyalkylénés et leurs sels, les acides alkyl(C6-C24) amido éther carboxyliques polyoxyalkylénés et leurs sels, en particulier ceux comportant de 2 à 50 groupements oxyde d'alkylène en particulier d'éthylène tels que les composés proposés par la société KAO sous les dénominations AKYPO, les acyl(C6-C24) sarcosinates et leurs sels, les acyl(C6-C24)lactylates et leurs sels et les acyl(C6-C24) glutamates. On peut également utiliser les esters d'alkyl(C6-C24)polyglycosides carboxyliques tels que les alkylglucoside acétates, les alkylglucoside citrates et les alkylpolyglycoside tartrate. De tels produits sont notamment vendus sous les dénominations de Eucarol APG/EC et Eucarol APG/ET par la société Lamberti et Plantapon LGC SORB par la société Cognis.

Les sels sont en particulier choisis parmi les sels alcalins, notamment de sodium, les sels d'ammonium, les sels d'amines, les sels d'aminoalcools tels la triéthanolamine ou la monoéthanolamine et les sels de magnésium.

On utilise de préférence les acyl(C6-C24) sarcosinates et leurs sels.

De manière plus spécifique, la composition peut comprendre du lauroyl sarcosinate de sodium, notamment vendu par la société Seppic sous le nom commercial Oramix^{®} L30.

La quantité de lauroyl sarcosinate de sodium peut être comprise entre 0,1 et 5 % en poids, de préférence entre 0,3 et 3 % en poids, par rapport au poids total de la composition.

Les tensioactifs non ioniques alkyl(poly) glucosides peuvent être en particulier le décylglucoside, le laurylglucoside, le cocoylglucoside ou le capryl/caprylyl glucoside. La composition selon l'invention comprend de préférence du capryl/caprylyl glucoside.

De manière plus spécifique, la composition peut comprendre du capryl/caprylyl glucoside, notamment vendu par la société Seppic sous le nom commercial Oramix^{®}

CG110. La quantité de capryl/caprylyl glucoside peut être comprise entre 0,1 et 5% en poids, de préférence entre 0,3 et 3 % en poids, par rapport au poids total de la composition.

De préférence, le tensioactif est choisi parmi le lauroyl sarcosinate de sodium, le capryl/capramidopropyl bétaïne, le caprylyl/capryl glucoside ou leur mélange.

Avantageusement, le tensioactif est présent dans la composition selon l'invention dans des quantités allant de de 0,1 à 10 % en poids, par rapport au poids total de la composition, de préférence de 0,1 à 5 % en poids, et plus particulièrement allant de 0,3 à 3 % en poids.

La composition selon l'invention peut comprendre d'autres composés comme les gommes de guar modifiées ou non. Plus particulièrement, la composition selon l'invention comprend des gommes de galactomannane cationiques (qui sont décrites plus particulièrement dans les brevets US3589578 et US4031307). On peut citer les gommes de guar comprenant des groupements cationiques trialkylammonium. On utilise par exemple des gommes de guar modifiées par un sel (par exemple un chlorure) de 2,3-époxypropyl triméthylammonium. De tels produits sont commercialisés notamment sous la dénomination JAGUAR^{®} EXCEL par la société Solvay ou SACI-CFPA. Les gommes de guar modifiées ou non sont présentes dans la composition avantageusement en des quantités comprises entre 0,05 et 0,2 % en poids, de préférence entre 0,05 et 0,15 % en poids, et mieux entre 0,08 et 0,13 % en poids, par rapport au poids total de la composition

Les compositions selon l'invention peuvent en outre comprendre des agents bénéfiques pour la peau ou le pelage. Ces ingrédients sont généralement introduits dans la composition pour améliorer les qualités du toucher de la fibre kératinique du pelage ou de la peau ou encore favoriser l'hydratation de cette dernière. Les polymères cationiques, les silicones et les émollients, comme les polyols, constituent dans ce cadre les ingrédients les plus couramment utilisés.

Avantageusement, la composition selon l'invention peut comprendre un ou plusieurs ingrédients choisis parmi le digluconate de chlorhexidine, la niacinamide, des extraits végétaux, tels que l'extrait d'avoine, l'extrait de *Punica granatum*, et un de leurs mélanges.

La composition selon l'invention peut comprendre en outre d'autres composés, en particulier choisis parmi des saccharides, des oligosaccharides, des polysaccharides hydrolysés ou non, modifiés ou non, des acides aminés, des oligopeptides, des peptides, des protéines hydrolysées ou non, modifiées ou non, des acides et alcools gras ramifiés ou non, des huiles ou des cires animales, végétales ou minérales, des céramides et des pseudo-céramides, des acides organiques hydroxylés, des antioxydants, des agents anti-radicaux libres, des polymères cationiques, anioniques ou non ioniques, solubles ou dispersés, ou un mélange de ces composés.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas ou substantiellement pas, altérées par la ou les adjonctions envisagées.

Ces additifs sont, éventuellement, présents dans la composition selon l'invention dans des proportions pouvant aller de 0, 00001 à 30 % en poids par rapport au poids total de la composition. La quantité précise de chaque additif est déterminée facilement par l'homme du métier selon sa nature et sa fonction.

Avantageusement, la composition selon l'invention est une composition aqueuse. Elle peut comprendre de l'eau en une teneur allant de 50 à 98 % en poids, par rapport au poids total de la composition, de préférence allant de 60 à 98 % en poids, préférentiellement allant de 70 à 98 % en poids.

La composition peut être sous la forme d'une lotion, d'un gel, d'une crème, d'une mousse. La composition selon l'invention est avantageusement sous forme de mousse, en particulier lorsqu'elle comprend le ou les tensioactif(s) décrit(s) précédemment. La mousse peut être formée à partir d'un mélange d'air ou de gaz propulseur avec la composition décrite précédemment.

La composition, avant sa distribution, peut être conditionnée dans un distributeur de moussse permettant la distribution de ladite composition sous forme de mousse.

Le distributeur peut être un distributeur aérosol. Celui-ci est généralement constitué d'un récipient en métal ou en verre ou en matière plastique, ledit réciptient étant fermé hermétiquement et pourvu d'un dispositif de prélèvement permettant la sortie du produit sous forme de mousse. Le dispositif de prélèvement comprend généralement un tube plongeur, une valve et un diffuseur muni d'une buse. Le distributeur comprend la composition selon l'invention et un gaz propulseur. Le gaz propulseur peut être choisi parmi le dioxyde d'azote, l'azote, le diméthyléther, le butane, l'isobutane, le propane, le pentane , et leurs mélanges.

Selon une autre variante de réalisation, le distributeur peut être non aérosol de type pompe. La compositon peut être distribuée à partir d'un récipient au moyen d'une pompe mécanique reliée à une tête de distribution, le passage de la composition dans la tête de distribution la transformant en mousse au plus tard au niveau de l'orifice de sortie de ladite tête.

Un autre objet de l'invention concerne un dispositif de traitement vétérinaire de la peau et/ou du pelage d'un mammifère non-humain comprenant :
- une composition contenant le tensioactif telle que décrite précédemment ; et
- un distributeur de mousse permettant la distribution de ladite composition sous forme d'une mousse.

L'invention a également pour objet un procédé non-thérapeutique pour traiter la peau et/ou le pelage d'un mammifère non-humain, et en particulier un chien ou un chat, en appliquant la composition de la présente invention sur la peau et/ou le pelage dudit mammifère. L'invention a également pour objet la composition de la présente invention pour une utilisation dans le traitement de la peau et/ou le pelage d'un mammifère non-humain, et en particulier d'un chien ou d'un chat.

Les compositions selon l'invention sont mises en oeuvre par simple application sur le mammifère non humain, éventuellement par massage ou friction avec les mains. La composition appliquée n'a pas besoin d'être rincée à l'eau.

Le procédé ou l'utilisation selon l'invention permet également de ne pas être irritant, voire de diminuer l'irritation de la peau, ce qui diminue le stress et donne un effet de bien-être au mammifère non-humain, en particulier le chien ou le chat.

Les compositions de type non rincée selon l'invention pour des mammifères non humains et en particulier des chiens et des chats permettent de préserver, voire améliorer, la fonction barrière de la peau et/ou du pelage. En particulier, la peau traitée est souple et hydratée durablement, et présente une bonne imperméabilité. La composition appliquée confère un bon renfort de la barrière mécanique de la peau ainsi qu'un effet hydratant durable, rémanent. Les compositions selon l'invention pour des mammifères non humains et en particulier des chiens et des chats permettent également de diminuer la présence de bactéries pathogènes ou leur développement ou de réduire et/ou prévenir la formation et/ou l'adhésion de biofilms de bactéries pathogènes, les bactéries pathogènes étant en particulier *Staphylococcus aureus* et/ou *Staphylococcus pseudintermedius.*

Les compositions selon l'invention permettent également de préserver, voire améliorer, l'aspect soyeux du pelage.

De plus, l'application de la composition non rincée sur la peau ou le pelage permet une action durable de la composition appliquée.

Par ailleurs, lorsque la composition est sous forme de mousse, elle s'applique facilement sur la surface de la peau ou du pelage, notamment par massage qui apporte un effet relaxant. Cette facilité d'application permet un usage fréquent de la composition qui peut être appliquée sur le mammifère plusieurs fois par semaine.

Un autre objet de l'invention concerne une composition telle que définie ci-dessus pour son utilisation pour réduire et/ou prévenir la formation et/ou l'adhésion de biofilms de bactéries pathogènes ou pour diminuer la présence sur la peau ou le pelage de bactéries pathogènes, telles que *Staphylococcus aureus* et/ou *Staphylococcus pseudintermedius*, chez un mammifère non-humain, en particulier le chien ou chat.

Ainsi, une composition telle que définie pour son utilisation pour réduire et/ou prévenir la formation et/ou l'adhésion de biofilms de bactéries pathogènes ou pour diminuer la présence de bactéries pathogènes peut être réalisée par l'application d'une composition telle que définie ci-dessus.

Selon un mode particulier de l'invention, le mammifère non-humain est choisi parmi les félidés, les canidés et/ou les équidés, et en particulier les chiens et les chats.

Le terme « environ » sera compris par l'homme du métier et peut varier dans une certaine mesure selon le contexte dans lequel il est utilisé. Si certaines utilisations de ce terme ne sont pas claires pour l'homme du métier en fonction du contexte, « environ » signifie plus ou moins 10 %, de préférence plus ou moins 5 % de la valeur indiquée.

Les valeurs indiquées dans les intervalles spécifiées dans le texte doivent être incluses, sauf mention contraire.

Selon l'invention, l'expression "comprend (s)" ou "comprenant" (et d'autres termes comparables, par exemple "contenant" et "y compris") est "ouverte" et peut être interprétée de manière générale de telle sorte que les caractéristiques spécifiquement mentionnées et toutes les caractéristiques optionnelles, supplémentaires et non spécifiées sont incluses. Selon des modes de réalisation spécifiques, elle peut également être interprétée comme une expression "consistant essentiellement en" dans laquelle sont incluses les caractéristiques spécifiées et toute caractéristique optionnelle, supplémentaire ou non spécifiée qui n'affecte pas matériellement la ou les caractéristiques fondamentales et nouvelles de l'invention revendiquée, ou comme une expression "consistant en" où seules les caractéristiques spécifiées sont incluses, sauf indication contraire.

Selon l'invention le terme « au moins un » signifie « un », « deux », trois », ou plusieurs.

D'autres aspects et avantages de l'invention apparaîtront à la lecture des exemples qui suivent, et qui doivent être considérés comme illustratifs et non limitatifs.

Les pourcentages indiqués sont en poids sauf mention contraire.

### EXEMPLES

Les compositions suivantes des tableaux 1 à 3 sont préparées selon les méthodes classiques de préparation de compositions aqueuses. Elles sont conditionnées dans un flacon pompe qui distribue la composition sous forme de mousse par foisonnement avec l'air.

Ces compositions présentent de bonnes propriétés d'application sur le pelage du chien et du chat, notamment par massage relaxant facilitant un usage fréquent, par exemple 3 fois par semaine. La composition appliquée est en contact direct avec la peau de façon plus durable. Elles permettent d'améliorer la fonction barrière, de restaurer la balance microbienne, et en particulier de diminuer la présence de bactéries pathogènes chez le chien ou le chat, et en particulier de limiter la formation ou l'adhésion de biofilm. Les bactéries pathogènes sont en particulier le *Staphylococcus aureus* et/ou *Staphylococcus pseudintermedius.*

**Tableau 1**

| **Composé Nom commercial (société)** | **Composé Dénomination INCI** | **% Composé dans composition** |
|---|---|---|
| Eau | AQUA | QS 100 |
| | CONSERVATEUR | qs |
| | PANTHENOL | 0,5 |
| Tego^{®} betaïne 810 (Evonik) | CAPRYLAMIDOPROPYL BETAINE / CAPRAMIDOPROPYL BETAINE en solution aqueuse à 35,5 % MA | 4 |
| Jaguar^{®} Excel (Solvay) | GUAR HYDROXYPROPYLTRIMONIUM CHLORIDE à 95 % MA avec de l'eau | 0,1 |
| Ophytrium (Ceva) | OPHIOPOGON JAPONICUS ROOT EXTRACT à 71 % MA avec maltodextrine | 0,75 |
| Pentavitin^{®} (DSM) | SACCHARIDE ISOMERATE en solution aqueuse à 50 % MA | 1 |

**Tableau 2**

| **Composé Nom commercial (société)** | **Composé Dénomination INCI** | **% Composé dans composition** |
|---|---|---|
| Eau | AQUA | QS 100 |
| | CHLOREXIDINE DIGLUCONATE | 3 |
| | CONSERVATEUR | qs |
| | PANTHENOL | 0,5 |
| Oramix^{®} CG110 (Seppic) | CAPRYLYL / CAPRYL GLUCOSIDE (en solution aqueuse à 60 % MA) | 2 |
| Jaguar^{®} Excel (Solvay) | GUAR HYDROXYPROPYLTRIMONIUM CHLORIDE à 95 % MA avec de l'eau | 0,1 |
| Ophytrium (Ceva) | OPHIOPOGON JAPONICUS ROOT EXTRACT à 74 % MA avec maltodextrine | 0,5 |
| Pentavitin^{®} (DSM) | SACCHARIDE ISOMERATE en solution aqueuse à 50 % MA | 0,1 |

**Tableau 3**

| **Composé Nom commercial (société)** | **Composé Dénomination INCI** | **% Composé dans composition** |
|---|---|---|
| Eau | AQUA | QS 100 |
| | CONSERVATEUR | qs |
| | PANTHENOL | 0,5 |
| Oramix^{®} L30 (Seppic) | SODIUM LAUROYL SARCOSINATE (en solution aqueuse à 30 % MA) | 2 |
| Ophytrium (Ceva) | OPHIOPOGON JAPONICUS ROOT EXTRACT à 74 % MA avec maltodextrine | 0,5 |
| Pentavitin^{®} (DSM) | SACCHARIDE ISOMERATE en solution aqueuse à 50 % MA | 1 |

## Revendications

1. Composition vétérinaire de type non rincée comprenant un actif obtenu à partir *d'Ophiopogon japonicus* , du panthénol, et du saccharide isomerate.

2. Composition vétérinaire selon la revendication 1, comprenant au moins 0,1% en poids de principe actif obtenu à partir *d'Ophiopogon japonicus,* préférentiellement entre 0.1 et 1% en poids, par rapport au poids total de la composition, et plus particulièrement entre 0,2 et 0,6 % en poids, par rapport au poids total de la composition.

3. Composition vétérinaire selon la revendication 1 ou 2, comprenant au moins 0,1 % en poids de panthénol, par rapport au poids total de la composition, préférentiellement de 0,1 à 1 % en poids, et plus particulièrement de 0,3 à 0,8 % en poids.

4. Composition vétérinaire selon l'une quelconque des revendications 1 à 3, comprenant au moins 0,1 % en poids de saccharide isomerate, par rapport au poids total de la composition, préférentiellement entre 0,1 et 1 % en poids, et plus particulièrement entre 0,3 et 0,9 % en poids.

5. Composition vétérinaire selon l'une quelconque des revendications précédentes, dans laquelle la composition est sous la forme choisie parmi une lotion, un gel, une crème, une mousse.

6. Composition vétérinaire selon l'une quelconque des revendications précédentes, dans laquelle la composition comprend un ou plusieurs tensioactif(s) choisi(s) parmi les tensioactifs amphotères de type bétaïne, les tensioactifs anioniques carboxyliques, les tensioactifs non ioniques alkyl(poly)glucosides, et leurs mélanges.

7. Composition vétérinaire selon la revendication 6, dans laquelle le tensioactif est choisi parmi le lauryl sarcosinate de sodium, le caprylamidopropyl bétaïne, le capryl glucoside ou leurs mélanges.

8. Composition vétérinaire selon la revendication 6 ou 7, dans laquelle le tensioactif est présent dans des quantités allant de de 0,1 à 10 % en poids, par rapport au poids total de la composition, de préférence de 0,1 à 5 % en poids, et plus particulièrement allant de 0,3 à 3 % en poids.

9. Composition vétérinaire selon l'une quelconque des revendications 1 à 8, dans laquelle la composition comprend en outre des gommes de galactomannane cationiques.

10. Composition vétérinaire selon l'une quelconque des revendications 1 à 9, dans laquelle la composition se présente sous forme de mousse.

11. Procédé non thérapeutique pour traiter la peau et/ou le pelage d'un mammifère non-humain, et en particulier un chien ou un chat, en appliquant la composition selon l'une des revendications 1 à 10 sur la peau et/ou le pelage dudit mammifère.

12. Composition vétérinaire selon l'une des revendications 1 à 10, pour son utilisation pour réduire et/ou prévenir la formation et/ou l'adhésion de biofilms de bactéries pathogènes ou pour diminuer la présence sur la peau ou le pelage de bactéries pathogènes, chez un mammifère non-humain, en particulier le chien ou chat.

13. Composition vétérinaire pour son utilisation selon la revendication 12, **caractérisée en ce que** les bactéries pathogènes sont choisies parmi le *Staphylococcus aureus* et/ou *Staphylococcus pseudintermedius.*

14. Dispositif de traitement vétérinaire de la peau et/ou du pelage d'un mammifère non-humain comprenant :
- une composition vétérinaire selon l'une des revendications 6 à 9 ; et
- un distributeur de mousse permettant la distribution de ladite composition sous forme d'une mousse.

## Patentansprüche

1. Veterinärmedizinische Zusammensetzung einer nicht gespülten Art, umfassend einen Wirkstoff, der aus *Ophiopogon japonicus*, Panthenol und Saccharidisomerat erhalten wird.

2. Veterinärmedizinische Zusammensetzung nach Anspruch 1, umfassend zu mindestens 0,1 Gew.-%, vorzugsweise zu zwischen 0,1 und 1 Gew.-%, bezogen auf das Gesamtgewicht der Zusammensetzung, und insbesondere zu zwischen 0,2 und 0,6 Gew.-%, bezogen auf das Gesamtgewicht der Zusammensetzung, den natürlichen Wirkstoff, der aus *Ophiopogon japonicus* erhalten wird.

3. Veterinärmedizinische Zusammensetzung nach Anspruch 1 oder 2, umfassend zu mindestens 0,1 Gew.-%, vorzugsweise zu 0,1 bis 1 Gew.-% und insbesondere zu 0,3 bis 0,8 Gew.-%, Panthenol, bezogen auf das Gesamtgewicht der Zusammensetzung.

4. Veterinärmedizinische Zusammensetzung nach einem der Ansprüche 1 bis 3, umfassend zu mindestens 0,1 Gew.-%, vorzugsweise zu zwischen 0,1 und 1 Gew.-% und insbesondere zu zwischen 0,3 und 0,9 Gew.-%, Saccharidisomat, bezogen auf das Gesamtgewicht der Zusammensetzung.

5. Veterinärmedizinische Zusammensetzung nach einem der vorstehenden Ansprüche, wobei die Zusammensetzung in der Form vorliegt, die aus einer Lotion, einem Gel, einer Creme, einem Schaum ausgewählt ist.

6. Veterinärmedizinische Zusammensetzung nach einem der vorstehenden Ansprüche, wobei die Zusammensetzung ein oder mehrere Tensid(e) umfasst, die aus amphoteren Tensiden der Betainart, anionischen Carbontensiden, nichtionischen Alkyl(poly)glucosidtensiden und Mischungen davon ausgewählt sind.

7. Veterinärmedizinische Zusammensetzung nach Anspruch 6, wobei das Tensid aus Natriumlaurylsarcosinat, Caprylamidopropylbetain, Caprylglucosid oder Mischungen davon ausgewählt ist.

8. Veterinärmedizinische Zusammensetzung nach Anspruch 6 oder 7, wobei das Tensid in Mengen von 0,1 bis 10 Gew.-%, vorzugsweise 0,1 bis 5 Gew.-% und insbesondere von 0,3 bis 3 Gew.-%, bezogen auf das Gesamtgewicht der Zusammensetzung, vorhanden ist.

9. Veterinärmedizinische Zusammensetzung nach einem der Ansprüche 1 bis 8, wobei die Zusammensetzung ferner kationische Galactomannangummis umfasst.

10. Veterinärmedizinische Zusammensetzung nach einem der Ansprüche 1 bis 9, wobei die Zusammensetzung in Form eines Schaums vorliegt.

11. Nichttherapeutisches Verfahren zum Behandeln der Haut und/oder des Fells eines nichtmenschlichen Säugetiers, und vor allem eines Hundes oder einer Katze, durch Auftragen der Zusammensetzung nach einem der Ansprüche 1 bis 10 auf die Haut und/oder das Fell des Säugetiers.

12. Veterinärmedizinische Zusammensetzung nach einem der Ansprüche 1 bis 10 für die Verwendung zum Verringern und/oder Verhindern der Ausbildung und/oder der Anhaftung von Biofilmen pathogener Bakterien oder zum Verringern des Vorhandenseins pathogener Bakterien auf der Haut oder dem Fell bei einem nichtmenschlichen Säugetier, vor allem dem Hund oder der Katze.

13. Veterinärmedizinische Zusammensetzung für die Verwendung nach Anspruch 12, **dadurch gekennzeichnet, dass** die pathogenen Bakterien aus *Staphylococcus aureus* und/oder *Staphylococcus pseudintermedius* ausgewählt sind.

14. Vorrichtung für die veterinärmedizinische Behandlung der Haut und/oder des Fells eines nichtmenschlichen Säugetiers, umfassend:
- eine veterinärmedizinische Zusammensetzung nach einem der Ansprüche 6 bis 9; und
- einen Schaumspender, der das Spenden der Zusammensetzung in Form eines Schaums ermöglicht.

## Claims

1. No-rinse veterinary composition comprising an active ingredient obtained from *Ophiopogon japonicus*, panthenol, and saccharide isomerate.

2. Veterinary composition according to claim 1, comprising at least 0.1 wt.% of active substance obtained from *Ophiopogon japonicus,* preferably between 0.1 and 1 wt.%, based on the total weight of the composition, and more particularly between 0.2 and 0.6 wt.%, based on the total weight of the composition.

3. Veterinary composition according to claim 1 or 2, comprising at least 0.1 wt.% of panthenol, based on the total weight of the composition, preferably 0.1 to 1 wt.%, and more particularly 0.3 to 0.8 wt.%.

4. Veterinary composition according to any of claims 1 to 3, comprising at least 0.1 wt.% of saccharide isomerate, based on the total weight of the composition, preferably between 0.1 and 1 wt.%, and more particularly between 0.3 and 0.9 wt.%.

5. Veterinary composition according to any of the preceding claims, wherein the composition is in the form selected from a lotion, gel, cream or foam.

6. Veterinary composition according to any of the preceding claims, wherein the composition comprises one or more surfactants selected from betaine amphoteric surfactants, carboxylic anionic surfactants, alkyl(poly)glucoside nonionic surfactants, and mixtures thereof.

7. Veterinary composition according to claim 6, wherein the surfactant is selected from sodium lauryl sarcosinate, caprylamidopropyl betaine, capryl glucoside or mixtures thereof.

8. Veterinary composition according to claim 6 or 7, wherein the surfactant is present in quantities ranging from 0.1 to 10 wt.%, based on the total weight of the composition, preferably from 0.1 to 5 wt.%, and more particularly ranging from 0.3 to 3 wt.%.

9. Veterinary composition according to any of claims 1 to 8, wherein the composition further comprises cationic galactomannan gums.

10. Veterinary composition according to any of claims 1 to 9, wherein the composition is in the form of a foam.

11. Non-therapeutic method for treating the skin and/or coat of a non-human mammal, and in particular a dog or cat, by applying the composition according to any of claims 1 to 10 to the skin and/or coat of said mammal.

12. Veterinary composition according to any of claims 1 to 10, for use in reducing and/or preventing the formation and/or adhesion of biofilms of pathogenic bacteria or in reducing the presence of pathogenic bacteria on the skin or coat, in a non-human mammal, in particular a dog or cat.

13. Veterinary composition for use according to claim 12, **characterized in that** the pathogenic bacteria are selected from *Staphylococcus aureus* and/or *Staphylococcus pseudintermedius.*

14. Device for veterinary treatment of the skin and/or coat of a non-human mammal, comprising:
- a veterinary composition according to any of claims 6 to 9; and
- a foam dispenser for dispensing said composition in the form of a foam.
